# EUROPEAN PATENT SPECIFICATION

(11) **EP 3 072 850 B1**
(45) Date of publication and mention of the grant of the patent: **11.09.2019**
(21) Application number: 14863224.3
(22) Date of filing: 19.11.2014
(51) Int. Cl.: C01B 32/194, C07C 335/16

(54) **METHOD FOR PREPARING FUNCTIONALIZED GRAPHENE**
VERFAHREN ZUR HERSTELLUNG VON FUNKTIONALISIERTEM GRAPHEN
PROCÉDÉ DE PRÉPARATION DE GRAPHÈNE FONCTIONNALISÉ

(30) Priority: 19.11.2013 KR 20130141006
(43) Date of publication of application: 28.09.2016
(73) Proprietor: Hanwha Chemical Corporation, Seoul 04541 (KR)
(72) Inventor: LEE, Jin Seo, Yongin-si Gyeonggi-do 446-569 (KR); DO, Seung Hoe, Yuseong-gu Daejeon 305-768 (KR); JEON, Seong Yun, Daejeon 305-707 (KR); HAN, Gi Woo, Daejeon 305-251 (KR); KONG, Jung Ho, Daejeon 305-741 (KR)
(74) Representative: Alt, Michael
(86) International application number: PCT/KR2014/011143
(87) International publication number: WO 2015/076565

(56) References cited:
- EP-A1- 3 037 384
- CN-A- 103 172 057
- JP-A- 2013 006 732
- KR-B1- 101 001 385
- KR-B1- 101 034 579
- KR-B1- 101 034 580
- KR-B1- 101 264 147
- SU YUEZENG ET AL: "Low-temperature synthesis of nitrogen/sulfur co-doped three-dimensional graphene frameworks as efficient metal-free electrocatalyst for oxygen reduction reaction", CARBON, vol. 62, 13 June 2013 (2013-06-13), pages 296-301, XP028678191, ISSN: 0008-6223, DOI: 10.1016/J.CARBON.2013.05.067
- Anonymous: "Ammonium thiocyanate", Wikipedia, 9 May 2013 (2013-05-09), pages 1-3, XP055356718, Retrieved from the Internet: URL:https://web.archive.org/web/2013050902 2147/http://en.wikipedia.org/wiki/Ammonium _thiocyanate [retrieved on 2017-03-21]
- WEI AI ET AL: "A Novel Graphene-Polysulfide Anode Material for High-Performance Lithium-Ion Batteries", SCIENTIFIC REPORTS, vol. 3, 1 August 2013 (2013-08-01), pages 1-5, XP055356834, DOI: 10.1038/srep02341 -& Wei Ai ET AL: "Supporting Information - A Novel Graphene-Polysulfide Anode Material for High-Performance Lithium-Ion Batteries", , 1 August 2013 (2013-08-01), pages 1-11, XP055356841, Retrieved from the Internet: URL:http://www.nature.com/article-assets/n pg/srep/2013/130801/srep02341/extref/srep0 2341-s1.doc [retrieved on 2017-03-21]
- HUI GAO ET AL: "Paper;Synthesis of S-doped graphene by liquid precursor;Synthesis of S-doped graphene by liquid precursor", NANOTECHNOLOGY, IOP, BRISTOL, GB, vol. 23, no. 27, 19 June 2012 (2012-06-19) , page 275605, XP020225618, ISSN: 0957-4484, DOI: 10.1088/0957-4484/23/27/275605

## Description

### [Technical Field]

The present invention relates to a method as defined in the claims for preparing a functionalized graphene, and more specifically, to a method for preparing a functionalized graphene having excellent electrical and thermal conductivity properties, and a barrier property, using a fluid in a subcritical condition or a supercritical condition and a functional compound. Also described is an apparatus for preparing a functionalized graphene and a functionalized graphene.

### [Background Art]

Graphene is a material in which carbon atoms of graphite which is a three-dimensional structured carbon allotrope naturally present in the natural world are arranged in a hexagonal plane structure which is a two-dimensional sheet form. Carbon atoms of the graphene form a sp² bond, and have a plane sheet form in a single atom thickness.

The graphene has significantly excellent electric conductivity and thermal conductivity, and physical properties such as excellent mechanical strength, flexibility, elasticity, quantified transparency depending on thickness, high specific surface area, and the like, may be explained by specific bonding structure of atoms present in the graphene. Three of four peripheral electrons of the carbons configuring the graphene form a sp² hybrid orbital to have a sigma bond, and remaining one electron and the surrounding carbon atoms form a pi bond to provide a hexagonal two-dimensional structure. Therefore, the graphene has a band structure which is different from other carbon allotropes, and does not have a band gap to exhibit excellent electric conductivity; however, the graphene is a semi-metal material in which state density of electrons at the Fermi level is 0, and therefore, may easily change electrical properties depending on whether or not it is doped.

Accordingly, since the graphene may be variously applied to automobile, energy, aerospace, construction, pharmaceutical, and steel fields as well as various electric electronic fields such as next-generation materials, capacitors, electromagnetic shielding materials, sensors, displays, and the like, which are replaceable for silicon electric electronic materials, research into a technology of utilizing the graphene in various fields has been largely conducted.

As a method for preparing the graphene, a scotch-tape method or a peel off method for exfoliating a graphene single layer from the graphite sheet using an adhesive tape, chemical vapor deposition, an epitaxial growth method by lamination on a silicon carbide substrate (SiC), thermal exfoliation of exfoliating the graphite by using heat, chemical oxidation and reduction, or the like, has been researched.

Among them, the chemical oxidation and reduction has advantages in that mass-production is possible, economical feasibility is provided, and various functional groups may be easily introduced into the graphene sheet. Meanwhile, in this method, reducing agents such as hydrazine, and the like, should be used for a deoxygenation reaction of graphene oxide, wherein most of these reducing agents are dangerous due to high corrosiveness, explosiveness, human toxicity, and the like, and the prepared graphene may include impurities, and the like, such that electric conductivity may be decreased. In addition, when materials such as a metal are intercalated between a layered structure for preventing restacking phenomenon of the graphene formed in forming the graphene (metal intercalation), size and distribution of particles to be intercalated (donor intercalant) are not uniform, such that it is difficult to prepare high quality graphene.

Further, when the functional group is introduced into a surface of the graphene in order to improve dispersibility and impart functionality of the graphene, separate reactions in various stages using general batch typed reactors should be processed; however, in this case, it is difficult for the functional group to be uniformly distributed on the surface of the graphene, and an amount of the functional group to be introduced is also difficult to be adjusted.

Therefore, research into a method for preparing a functionalized graphene having excellent physical properties such as dispersibility, functionality, electrical conductivity, and the like, even by economical, efficient, and low-risk processes, should be conducted.

### [Related Art Document]

(Patent Document 1) Korean Patent Publication No. KR 2012-0013914A (Patent Document 2) Korean Patent Publication No. KR 2012-0006458A (Patent Document 3) Korean Patent Publication No. KR 2012-0114317A

Su Yuezeng et al, "Low-temperature synthesis of nitrogen/sulfur co-doped three-dimensional graphene frameworks as efficient metal-free electrocatalyst for oxygen reduction reaction", CARBON, (20130613), vol. 62, doi:10.1016/J.CARBON.2013.05.067, ISSN 0008-6223, pages 296 - 301, discloses a one-pot hydrothermal approach towards three-dimensional nitrogen and sulfur co-doped graphene frameworks (N/S-GFs) employing graphene oxide and ammonium thiocyanate as the precursors.

EP 3 037 384 (A1) discloses a method for preparing a graphene including forming a mixed solution including a graphite oxide, a solvent, a first oxidizing agent; and a sulfur compound or a nitrogen compound; forming the graphene by reacting the mixed solution under a subcritical condition or a supercritical condition of the solvent; and recovering the graphene.

### [Disclosure]

### [Technical Problem]

The present invention has been made in an effort to provide a method and an apparatus for preparing a functionalized graphene having excellent physical properties such as dispersibility, functionality, electrical conductivity, and the like, even by economical and efficient and low-risk processes.

In addition, the present invention has been made in an effort to provide a functionalized graphene with a functional group including a sulfur atom.

### [Technical Solution]

An exemplary embodiment of the present invention provides a method for preparing a functionalized graphene including: forming a mixture including a graphite oxide, water, and thiourea; pre-heating the mixture to 200 to 400°C; forming the functionalized graphene by reacting the mixture under a subcritical condition of the solvent; and recovering the functionalized graphene.

In addition, another exemplary embodiment provides an apparatus for preparing a functionalized graphene including: a mixing part forming a mixture including a graphite oxide, a solvent, and a functional compound including all of an amine group and a sulfur atom; a preheater pre-heating the mixture supplied from the mixing part; a reactor connected to a rear end of the preheater, and generating a reaction of the mixture under a subcritical condition or a supercritical condition of the solvent; a cooling down system connected to a rear end of the reactor and cooling down a product of the reaction; and product storing parts connected to a rear end of the cooling down system and recovering the functionalized graphene from the product.

Further, another exemplary embodiment provides a functionalized graphene with a functional group including a sulfur atom.

### [Advantageous Effects]

According to a method of the present invention and an apparatus for preparing a functionalized graphene, graphene oxide may be effectively reduced, and at the same time, a functionalized graphene in which a sulfur atom is introduced into a basal plane and an edge thereof may be prepared. Accordingly, the functionalized graphene capable of being utilized as materials throughout the industry, such as barrier materials, lightweight materials, energy, batteries, electronics, electrics, semiconductors, displays, home appliances, mobile phones, nano-industries, biotechnologies, polymer composites, metal composites, paints, pastes, inks, and the like, may be prepared. In addition, according to the present invention, since a deoxygenation reaction and a functionalization reaction of the graphene surface are simultaneously performed under a subcritical condition or a supercritical condition, high quality functionalized graphene having high degree of reduction, high exfoliation property, and high dispersibility due to the continuous process may be prepared.

### [Brief Description of Drawings]

FIG. 1 exemplarily shows an apparatus for preparing a functionalized graphene according to an exemplary embodiment.
FIG. 2 exemplarily shows an apparatus for preparing a functionalized graphene according to an exemplary embodiment.
FIG. 3 shows a Raman spectrum of functionalized graphenes obtained by Example 1 and Comparative Example 1 of the present invention.
FIG. 4 shows an infrared spectrum of the functionalized graphene obtained by Example 1 of the present invention.
FIG. 5 shows a scanning electron microscope (SEM) of the functionalized graphenes obtained by Example 1 and Comparative Example 1 of the present invention.
FIG. 6 shows a transmission electron microscope (TEM) of the functionalized graphenes obtained by Example 1 and Comparative Example 1 of the present invention.
FIG. 7 shows an XPS spectrum of a graphite oxide obtained by Preparation Example, the functionalized graphenes obtained by Examples 1 and 2 of the present invention.
FIG. 8 shows an XRD result of graphite, a graphite oxide obtained by Preparation Example, and the functionalized graphenes obtained by Example 1 and Comparative Example 1 of the present invention.

### [Best Mode]

A method for preparing a functionalized graphene of the present invention includes: forming a mixture including a graphite oxide, water, and thiourea; pre-heating the mixture to 200 to 400°C; forming the functionalized graphene by reacting the mixture under a subcritical condition of the solvent; and recovering the functionalized graphene.

In addition, an apparatus for preparing a functionalized graphene includes: a mixing part forming a mixture including a graphite oxide, a solvent, and a functional compound including all of an amine group and a sulfur atom; a preheater pre-heating the mixture supplied from the mixing part; a reactor connected to a rear end of the preheater, and generating a reaction of the mixture under a subcritical condition or a supercritical condition of the solvent; a cooling down system connected to a rear end of the reactor and cooling down a product of the reaction; and product storing parts connected to a rear end of the cooling down system and recovering the functionalized graphene from the product.

Further, a functionalized graphene of the present invention is functionalized with a functional group including a sulfur atom.

The terminology used herein is for the purpose of describing exemplary embodiments only and is not intended to be limiting of the present invention. As used herein, the singular forms are intended to include the plural forms as well, unless the context clearly indicates otherwise. It will be further understood that the terms "comprises", "provides" and/or "has", when used in this specification, specify the presence of stated features, integers, steps, or components, or combinations thereof, but do not previously exclude the presence or addition of one or more other features, integers, steps, components, or combinations thereof.

In addition, in the present invention, the description that each layer or element is formed "on" or "over" of each of the layers or elements means that each layer or element is directly formed over each of the layers or elements, or means that another layer or element is additionally formed between each layer, or on a subject or a substrate. Although the present invention can be modified variously and have several embodiments, the exemplary embodiments are illustrated in the accompanying drawings and will be described in detail in the detailed description. However, the present invention is not limited to the specific embodiments and should be construed as including all the changes, equivalents, and substitutions included in the spirit and scope of the present invention.

Hereinafter, the present invention will be described in detail.

The method for preparing a functionalized graphene according to an exemplary embodiment of the present invention includes: forming a mixture including a graphite oxide, water, and thiourea; pre-heating the mixture to 200 to 400°C; forming the functionalized graphene by reacting the mixture under a supercritical condition of the solvent; and recovering the functionalized graphene.

According to an exemplary embodiment of the present invention, the functional compound is thiourea. Also described herein is a functional compound including all of an amine group and a sulfur atom, which may be a compound selected from the group consisting of the following Chemical Formula 1, Chemical Formula 2, and Chemical Formula 3, or a mixture including at least one kind compound thereof: 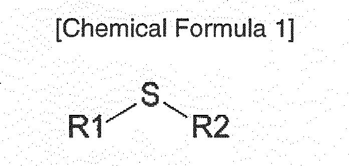 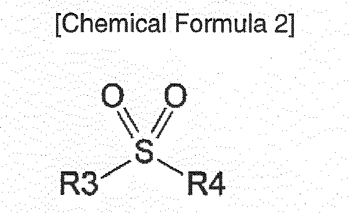 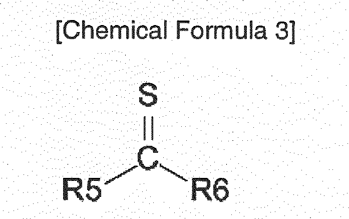
in Chemical Formulas 1, 2, and 3,
R1 to R6 are the same as or different from each other, and each independently hydrogen, an amino group, a C1-C20 alkyl group, a C3-C20 cycloalkyl group, a C1-C20 alkoxy group, a C1-C20 hydroxyalkyl group, a C2-C20 alkenyl group, a C6-C20 aryl group, a C7-C20 alkylaryl group, a C7-C20 arylalkyl group, a C1-C20 aminohydrocarbyl group, a C1-C20 hydrocarbylamino group, or a C1-C20 hydroxyalkylamino group, and
at least one of the R1 and R2, at least one of the R3 and R4, at least one of the R5 and R6 is an amino group, a C1-C20 aminohydrocarbyl group, a C1-C20 hydrocarbylamino group, or a C1-C20 hydroxyalkylamino group.

The amino group used herein means a functional group represented by -NH₂.

In addition, the hydrocarbylamino group used herein means primary, secondary, tertiary, quaternary hydrocarbylamino group or a hydrocarbylammonium group represented by -NH(R), -N(R)(R'), or -N(R)(R')(R")⁺ obtained by substituting at least one of hydrogen of nitrogen atoms in the amino group with an alkyl group.

Further, the hydroxyalkylamino group used herein means an group represented by -NH(ROH), -N(ROH)(R'OH), or -N(ROH)(R'OH)(R"OH)⁺ obtained by substituting at least one of hydrogen of nitrogen atoms in the amino group with a hydroxyalkyl group.

In addition, the aminohydrocarbyl group used herein means a functional group represented by -R-NH2, -R-NH(R'), -R-N(R')(R"), -R-NH3⁺, -R-NH2(R')⁺, or-R-NH(R')(R")⁺ obtained by substitution of the amino group or the hydrocarbylamino group in the middle or at the end of the hydrocarbyl group.

In the definition of the substituents, R, R', and R" are the same as or different from each other, and each independently a hydrocarbyl group.

The functional compound includes all of the amine group and the sulfur atom in a molecule. The amine group has a reduction property to help reduction of the graphene oxide in a deoxygenation reaction of the graphene oxide to be described below so that the reaction is effectively performed.

In addition, in the case of using the functional compound, the functional group having a sulfur atom is introduced into a surface or an edge of the graphene to be formed, such that the functionalized graphene having excellent dispersibility may be obtained. For example, the substituent such as an alkyl group, a cycloalkyl group, an alkenyl group, an aminoalkyl group, an alkylamino group, or the like, which may be included in the functional compound may be introduced into the surface or the edge of the graphene as a functional group together with the sulfur atom, and due to a steric hindrance effect of the substituent introduced into the surface or the edge of the graphene, restacking phenomenon in which the graphene separated from the graphite is restacked to the graphite may be prevented, such that the graphene may have improved quality.

Further, polarity of the surface of the graphene is capable of being adjusted by hydrophilic electron donating elements such as sulfur included in the functional group, and the like, and hydrophobic molecules, such that compatibility may be increased in accordance with the polarity of various matrixes (solution or solid), and therefore, the functionalized graphene having excellent dispersibility may be effectively prepared.

Meanwhile, since the functional compound has a property of preventing corrosion against metal materials, corrosion of the metal may be effectively prevented by using a barrier property of the prepared functionalized graphene.

Specific examples of the functional compound may include thiourea, thiourea dioxide, phenylthiourea, tolyl-3,3-dimethylthiourea, 1,3-dialkyloylthiourea, aminoundecanethiol, and the like.

The functionalized graphene formed in the forming of the functionalized graphene may be a functionalized graphene sheet, a functionalized graphene platelet, or a functionalized graphene nanoplatelet.

The functionalized graphene refers to graphene having a functional group on the surface or at the edge of the graphene, wherein the functional group may be introduced from the functional compound, and may include the sulfur atom. Specific examples thereof may include a sulfide group, an alkylsulfide group, an aminoalkylsulfide group, a sulfoxide group, an alkylsulfoxide group, an aminoalkylsulfoxide group, a sulfone group, an alkylsulfone group, an aminoalkylsulfone group, a thiol group, a thio group, and the like, but the present invention is not limited thereto.

The graphene sheet refers to a sheet-shaped carbon structure formed of a single layered structure separated from the graphite, and the graphene platelet or the graphene nanoplatelet means a carbon structure in which the graphene sheets are overlapped and agglomerated with each other, or a nanoscale carbon structure.

The preparation method may be more specifically explained in the following steps.

First, the mixture including the graphite oxide, the solvent, and the functional compound including all of the amine group and the sulfur atom is formed. The mixture may be a mixture in which the graphite oxide and the functional compound are dispersed in the solvent. Here, the graphite oxide dispersed in the solvent may have a single atom layer structure to a multiple atom layer structure in a flake shape.

In the graphite oxide, the functional group such as an epoxy group, a carboxyl group, a carbonyl group, a hydroxyl group, and the like, which are forms in which carbon, the main component of the graphite, is oxidized, may be present to have hydrophilic property, such that the graphite oxide may have excellent dispersibility to water which is the solvent, and may be easily dispersed into the solvent to be formed in a uniform mixture. Here, in order to effectively disperse the graphite oxide in the solvent, general dispersion methods using ultrasound, a homogenizer, and the like, may be used.

According to an exemplary embodiment of the present invention, the solvent is water and the functionalized graphene is formed by reacting the mixture under a supercritical condition of the solvent. Also described herein is that the solvent may include carbon dioxide, alcohol, or mixtures thereof. Water, carbon dioxide, alcohol, or mixtures thereof in the solvent may be subcritical water or supercritical water, subcritical carbon dioxide or supercritical carbon dioxide, or subcritical alcohol or supercritical alcohol under subcritical condition or supercritical condition, and may be reacted with the functional compound to form the functionalized graphene.

The alcohol is used without specific limitation as long as it is generally used for reduction of the graphite oxide in technical field that the present invention pertains, and the alcohol may include linear or branched about C1-C10 aliphatic alcohols, specifically, for example, methanol, ethanol, propanol, 2-propanol, butanol, 2-methylpropanol, 2-methylpropan-2-ol, pentanol, 2-pentanol, 3-pentanol, hexanol, 2-hexanol, 2-methyl-1-pentanol, methyl-1-pentanol, 4-methyl-1-pentanol, 2-methyl-2-pentanol, 3-methyl-2-pentanol, 4-methyl-2-pentanol, 2-methyl-3-pentanol, 3-methyl-3-pentanol, 2,2-dimethyl-1-butanol, 2,3-dimethyl-1-butanol, 2,3-dimethyl-2-butanol, 2-ethyl-1-butanol, 1-heptanol, 2-heptanol, 3-heptanol, 4-heptanol, or a mixture including at least one kind thereof, but the present invention is not limited thereto.

The graphite oxide included in the mixture may be formed by treating a graphite oxide precursor with an acid and an oxidizing agent, and in this case, the graphite oxide may be formed by Hummers method which is generally used.

The oxidizing agent, for example, peroxides such as permanganate, dichromate, chlorate, and the like, may be used without specific limitation, and when the graphite oxide precursor is treated with a strong acid such as sulfuric acid, nitric acid, hydrochloric acid, or the like, and an oxidizing agent such as permanganate, dichromate, chlorate, or the like, by Hummers method, the graphite oxide may be obtained. The prepared graphite oxide may have hydrophilic property by the functional group present in the graphite as described above, and the solvent may be easily penetrated between planes, such that exfoliation may be easily performed. Therefore, at the time of stirring a pre-mixture in the uniformed mixture form, the graphene oxide having a single atom layer structure exfoliated from the graphite oxide may be obtained.

The graphite oxide precursor may be used without specific limitation as long as it is a material in which carbon is a main component as well as general graphite. For example, graphene, graphite nanoplatelet, graphene nanoplatelet, expanded graphite or expandable graphite, diamond, fullerene, carbon black, activated carbon, charcoal, carbon nanoribbon, carbon nanowire, carbon nanoclay, carbon nanotube, carbon fiber or carbon nano fiber including pitch carbon fiber, and the like, carbon glass fiber, asphalt, or mixtures thereof, and the like, may be used.

According to an exemplary embodiment of the present invention, the solvent may be included in an amount of about 10 to about 100,000 parts by weight, preferably, about 1,000 to about 50,000 parts by weight, based on about 100 parts by weight of the graphite oxide. When the solvent is included in an amount more than about 100,000 parts by weight based on the graphite oxide, an amount of the graphene to be capable of being prepared in a unit time is extremely small, such that economical feasibility may be deteriorated, and when the solvent is included in an amount less than 10 parts by weight, the graphite oxide may not be effectively exfoliated, and the reaction with a subcritical fluid or a supercritical fluid to be described below (that is, the deoxygenation reaction and the functionalization reaction) may not be effectively performed, such that the functionalized graphene to be formed may have poor uniformity to deteriorate quality.

According to an exemplary embodiment of the present invention, the functional compound including all of the amine group and the sulfur atom may be included at about 0.1 to about 30 molar ratio, preferably about 0.1 to about 20 molar ratio, more preferably about 0.1 to about 10 molar ratio, with respect to oxygen included in the graphite oxide.

When the functional compound is included at a molar ratio less than about 0.1 with respect to oxygen included in the graphite oxide, reduction of the graphite oxide may not be effectively achieved, and a problem that the functional group is not sufficiently introduced into the surface and the edge of the functionalized graphene to be prepared may occur, and when the functional compound is included at a molar ratio more than about 30, the functional compound in an excessive amount may be used, such that the cost for the functional compound, and the cost for removing unreacted functional compound may be increased, which is not economical.

The mixture may be fed into the reactor at a high-pressure in order to perform the subsequent reaction under the subcritical condition or the supercritical condition. The pressure fed into the reactor is not specifically limited, but preferably, may be about 30atm to about 500atm.

The fed mixture is pre-heated to a temperature of about 200 to about 400°C by the pre-heating step. Through the pre-heating process, the reactor may be maintained at a predetermined temperature.

The pre-heated mixture may reach at the subcritical condition or the supercritical condition by a heating process and a pressing process. The solvent included in the mixture may be a subcritical fluid or a supercritical fluid under this condition, and when water, carbon dioxide, or alcohol is included in the solvent, each of water, carbon dioxide, or alcohol may reach at a subcritical water state or a supercritical water state, a subcritical carbon dioxide state or a supercritical carbon dioxide state, or a subcritical alcohol state or a supercritical alcohol state in this state.

The graphene oxide present as being exfoliated in the mixture may generate a reaction (a deoxygenation reaction) with the subcritical fluid or the supercritical fluid to be reduced, thereby forming the graphene. The deoxygenation reaction may be performed only by the reaction between the subcritical fluid or the supercritical fluid with the graphene oxide; however, the deoxygenation reaction may be more effectively promoted by the amine group of the functional compound included in the mixture. The amine group present in the functional compound is a reducing agent to be capable of reducing the graphene oxide, such that the deoxygenation reaction may be effectively generated, and at the same time, the surface of the graphene may be functionalized. As a result, the amine group helps effective preparation of the functionalized graphene. Through the reaction, the graphene oxide may be reduced into the graphene, and the functional group including the sulfur atom included in the functional compound may be introduced into the surface of the graphene or the edge of the graphene to prepare the functionalized graphene.

According to an exemplary embodiment of the present invention, a temperature of the subcritical condition or the supercritical condition may be about 25 to about 600°C, preferably, about 50 to about 500°C, and more preferably, about 200 to about 500°C. When the temperature is extremely low, reduced level of the subcritical fluid or the supercritical fluid is deteriorated, such that the deoxygenation reaction of the exfoliated graphene oxide may not be effectively performed, and when the temperature is extremely high, economical feasibility may be deteriorated due to the cost for maintaining high temperature condition.

According to an exemplary embodiment of the present invention, a pressure of the subcritical condition or the supercritical condition may be about 1 to about 500 atm, preferably, about 100 to about 500 atm. When the pressure is less than about 1atm, the reaction of the graphene oxide and the functional compound may not be effectively performed, and reduced level of the subcritical fluid or the supercritical fluid is deteriorated, such that the deoxygenation reaction of the exfoliated graphene oxide may not be effectively performed, and when the pressure is more than about 500 atm, economical feasibility may be deteriorated due to the cost for maintaining high-pressure condition.

Then, in the recovering of the formed functionalized graphene, the functionalized graphene formed by the reaction may be separated and recovered from the mixture including the solvent. The separating process may be performed by methods such as a drying method, centrifugation, filtration, and the like, of the mixture including the solvent, and the like, and any method for separating the prepared functionalized graphene from the mixture may be used without specific limitation.

According to an exemplary embodiment of the present invention, a process of high-pressure-filtering the formed functionalized graphene may be further included in the filtering process after the process of forming the functionalized graphene and before the process of recovering the functionalized graphene. The functionalized graphene with high purity may be more effectively separated by the filtering process at high-pressure in which the pressure of the reaction condition is not reduced as described above.

According to another exemplary embodiment of the present invention, a process of washing the formed functionalized graphene may be further included after the process of forming the functionalized graphene and before the process of recovering the functionalized graphene. Distilled water is high-pressure fed and supplied into a high-pressure filtering part used in the filtering process, such that the washing process may be continuously performed, and impurities such as the solvent, unreacted functional compound, side reaction materials, and the like, which may remain in the functionalized graphene may be effectively removed by the washing process.

Meanwhile, the apparatus for preparing a functionalized graphene according to an exemplary embodiment includes a mixing part forming a mixture including a graphite oxide, a solvent, and a functional compound including all of an amine group and a sulfur atom; a -preheater pre-heating the mixture supplied from the mixing part; a reactor connected to a rear end of the preheater, and generating a reaction of the mixture under a subcritical condition or a supercritical condition of the solvent; a cooling down system connected to a rear end of the reactor and cooling down a product of the reaction; and product storing parts connected to a rear end of the cooling down system and recovering the functionalized graphene from the product.

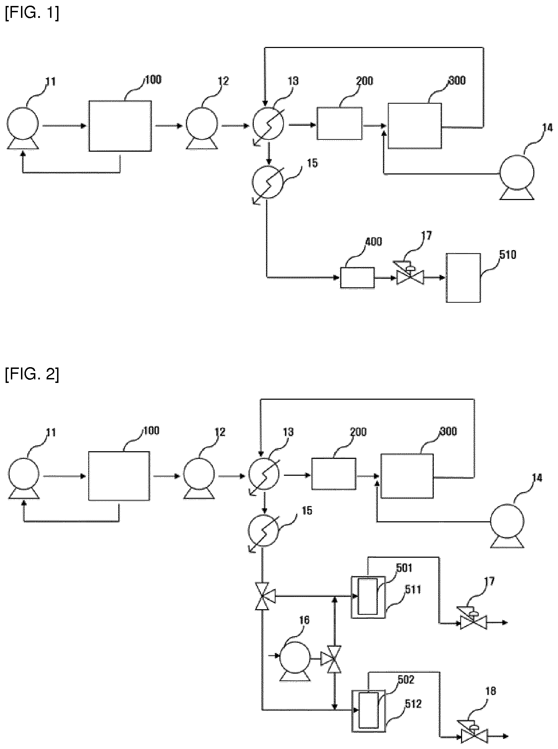

FIGS. 1 and 2 are views exemplarily showing an apparatus for preparing a functionalized graphene according to an exemplary embodiment.

Referring to FIGS. 1 and 2, the apparatus for preparing the functionalized graphene according to an exemplary embodiment includes: a mixing part 100 forming a mixture including a graphite oxide, a solvent, and a functional compound including all of an amine group and a sulfur atom; a preheater 200 pre-heating the mixture supplied from the mixing part; a reactor 300 connected to a rear end of the preheater, and generating a reaction of the mixture under a subcritical condition or a supercritical condition of the solvent; a cooling down system 15 connected to a rear end of the reactor and cooling down a product of the reaction; and product storing parts 510, 511, and 512 connected to a rear end of the cooling down system and recovering the functionalized graphene from the product. In addition, the apparatus may further include a cooling down and depressurizing system 400 provided between the cooling down system and the product storing part, and capable of cooling down and depressurizing the product at a temperature and a pressure appropriate for recovering the product.

For example, the reaction generated in the serial and continuous apparatus is as follows. The mixture is formed in the mixing part 100, the mixture is delivered through a high-pressure feeding pump 12 to the preheater 200. The mixture is pre-heated in the preheater 200 to a temperature of about 25 to about 500°C, preferably, about 50 to about 400°C, and more preferably, about 200 to about 400°C, and delivered to the reactor 300. In the reactor 300, the above-described reaction is performed in the method for preparing the functionalized graphene. The functionalized graphene formed by the reaction is delivered to the cooling down system 15, cooled down in the cooling down system 15 and/or cooling down and depressurizing system 400 at a temperature of about 20 to about 50°C, and recovered in the product storing parts 510, 511, and 512.

The apparatus for preparing the functionalized graphene may further include a circulation pump 11 connected to the mixing part 100, and circulating and mixing the graphite oxide, the solvent, and the functional compound including all of an amine group and a sulfur atom to form the mixture. The a pre-mixture mixed in the mixing part 100 is passed through the circulation pump 11 and returns to the mixing part to be uniformly mixed, and may be put into the preheater 200 in a state in which the mixture is more appropriate for the reaction.

According to another exemplary embodiment, the apparatus for preparing the functionalized graphene may further include the high-pressure feeding pump 12 provided between the mixing bath 100 and the-preheater 200, and supplying the mixture of the mixing bath to the preheater. The high-pressure feeding pump may supply the mixture of the mixing part to the preheater at the above-described pressure range of about 30atm to about 500atm.

Meanwhile, according to another exemplary embodiment of the present invention, the method for preparing the functionalized graphene may further include a functional compound feeding pump 14 connected to a front end of the reactor 300 and supplying the functional compound to the reactor. The functional compound may be supplied to the reactor 300 in a state in which the functional compound is mixed with the graphite compound in the mixing part 100 at first; however, may be directly supplied to the reactor 300 through the functional compound feeding pump 14.

According to another exemplary embodiment, the apparatus for preparing the functionalized graphene may further include a heat exchanger 13 provided between the mixing part 100 and the preheater 200, and connected to the rear end of the reactor 300 and a front end of the cooling down system 15. The heat exchanger 13 may deliver heat of the product obtained after the reaction is completed in the reactor 300 to the mixture of the-preheater 200. The product obtained after the reaction is completed in the reactor 300 is passed through the heat exchanger 13, and is cooled down at a temperature of about 150 to about 300°C while delivering heat to the preheater 200 through the heat exchanger 13, and at the same time, in the preheater 200, pre-heating may be performed by using the delivered heat. Energy required for cooling down the product and pre-heating the reactant may be reduced by heat-exchange generated by the heat exchanger 13.

According to an exemplary embodiment, the product storing parts 511 and 512 of the apparatus for preparing the functionalized graphene may further include filtering parts 501 and 502 filtering the product delivered from the cooling down system. The filtering parts 501 and 502 may include a high-pressure filtering part. The product cooled down after the reaction is completed in the reactor 300 is passed through the filtering parts 501 and 502 in a high-pressure state to achieve the high-pressure filtering process, and by the high-pressure filtering process in the filtering parts 501 and 502, functionalized graphene with high purity may be more effectively separated. The high-pressure filtering part may have separate pressure controlling systems 17 and 18 attached therewith, and filtering pressure may be appropriately controlled by the pressure controlling systems.

In addition, the filtering parts 501 and 502 may be connected to a distilled water feeding pump 16. The product may be washed by high-pressure injecting distilled water into the high-pressure filtering part through the distilled water feeding pump 16, and impurities such as the solvent, unreacted functional compound, side reaction materials, and the like, which may remain in the functionalized graphene may be effectively and easily removed by the washing process.

Meanwhile, according to another exemplary embodiment of the present invention, a functionalized graphene with a functional group including a sulfur atom is added. The functionalized graphene refers to graphene having the functional group on the surface or at the edge of the graphene, wherein the functional group is not specifically limited, but is introduced from the functional compound, and may include the sulfur atom. Specific examples thereof may include a sulfide group, an alkylsulfide group, an aminoalkylsulfide group, a sulfoxide group, an alkylsulfoxide group, an aminoalkylsulfoxide group, a sulfone group, an alkylsulfone group, an aminoalkylsulfone group, a functional group containing a thiol group, a functional group containing a thio group, and the like.

The functionalized graphene of the present invention may include about 0.1 to about 20 wt% of the sulfur atom, preferably, about 0.1 to about 15 wt% of the sulfur atom, and more preferably, about 1 to about 10 wt% of the sulfur atom when being measured by XPS. The sulfur atom having the weight ratio in the above-described range is appropriate for effect which is obtainable by introducing the functional group into the surface or the edge of the graphene as described above.

Meanwhile, the functionalized graphene shows a peak caused by 2p electron orbital of sulfur (S), that is, a characteristic peak at about 155 to about 175eV, and more preferably, about 160 to about 170eV, when being measured by XPS. The characteristic peak refers to a peak having intensity about ten times or more the background signal around the corresponding peak (white noise) to be easily differentiated by naked eyes, and the characteristic peak shown in about 155 to about 175eV, preferably, about 160 to about 170eV indicates binding energy by S2p of the sulfur atom, such that it may be confirmed that the functional group including the sulfur atom is introduced into the surface or the edge of the graphene.

The functionalized graphene may be prepared by the above-described method for preparing the functionalized graphene, such that more detailed description of the preparation method will be omitted.

In the functionalized graphene of the present invention, the restacking phenomenon in which the graphene separated from the graphite is restacked to the graphite may be prevented due to the steric hindrance effect of the functional group introduced into the surface or the edge of the graphene, such that the graphene may have increased quality, and polarity of the surface of the graphene is capable of being adjusted by hydrophilic electron donating elements and hydrophobic molecules, such that compatibility may be increased in accordance with the polarity of various matrixes (solution or solid), and therefore, excellent dispersibility may be shown. In addition, a thio compound has a property of preventing corrosion against a metal, such that corrosion of the metal may be effectively prevented by using the barrier property of the prepared functionalized graphene.

Further, the functionalized graphene is functionalized by high electron donating elements such as sulfur to increase electron and hole mobility, such that more improved electric conductivity may be shown as compared to graphene which is not functionalized.

Therefore, the functionalized graphene may be effectively used in various fields such as barrier materials, lightweight materials, energy, batteries, electronics, electrics, semiconductors, displays, home appliances, mobile phones, nano-industries, biotechnologies, polymer composites, metal composites, paints, pastes, inks, water treatment, waste-water treatment, antistatic materials, electrostatic dispersion materials, conductive materials, electromagnetic wave shielding materials, electromagnetic wave absorbers, radio frequency (RF) absorbers, materials for solar cell, electrode materials for dye-sensitized-solar-cell (DSSC), electrical device materials, electronic device materials, semiconductor device materials, photoelectric device materials, notebook component materials, computer component materials, mobile phone component materials, PDA component materials, PSP component materials, component materials for game machine, housing materials, transparent electrode materials, opaque electrode materials, field emission display (FED) materials, back light unit (BLU) materials, liquid crystal display (LCD) materials, plasma display panel (PDP) materials, light emitting diode (LED) materials, touch panel materials, electronic quotation board materials, billboard materials, display materials, heating elements, heat radiating elements, plating materials, catalysts, cocatalysts, oxidizing agents, reducing agents, automobile component materials, ship component materials, aircraft component materials, protective tape materials, adhesive materials, tray materials, clean room materials, transport component materials, flame retardant materials, antibacterial materials, metal composite materials, non-ferrous metal composite materials, steel plate materials, materials for medical devices, building materials, flooring materials, materials for wallpaper, light source component materials, lamp materials, optical instrument component materials, materials for fabricating fibers, materials for manufacturing clothing, materials for electric products, materials for manufacturing electronic products, materials for secondary battery such as cathode active materials for secondary battery, anode active materials for secondary battery, and conductor, fuel cell materials, solar cell materials, memory devices, and capacitor materials, and the like.

Hereinafter, functions and effects of the present invention will be described in detail by specific examples of the present invention.

### <Example>

### Preparation of Graphite Oxide

### [Preparation Example]

A graphite oxide was prepared by Hummers method.

10g of graphite and 5g of sodium nitrate were mixed and stirred in 2L of 3-neck round bottom flask at 0°C, and 250ml of 98% sulfuric acid was slowly and drop-wisely added. The mixture was slowly stirred for 2 hours while maintaining a temperature of 5°C. After 2 hours, 40g of potassium permanganate was slowly added while maintaining a temperature of 35°C. 500ml of distilled water was slowly and drop-wisely added while stirring the mixture at high speed, then, 36ml of 3% hydrogen peroxide aqueous solution was drop-wisely added, 275ml of 3% hydrochloric acid solution was drop-wisely added, and the obtained mixture was filtrated and washed with 900ml of 3% hydrochloric acid solution and then washed with 3L of distilled water. The washed filtering part cake was dried in vacuum oven at 40°C for 24 hours to obtain graphite oxide.

### Preparation of Functionalized Graphene

### [Example 1]

Graphene was prepared by using an apparatus shown in FIG. 2.

First, 5g of the graphite oxide prepared by Preparation Example and 972.7g of distilled water were put into the mixing part 100, and 23.3g(0.3M) of thiourea (S=C(NH₂)₂) was added while being stirred, then circulated by the circulation pump 11 to prepare a mixture containing the thiourea. The mixture was put into the preheater 200 by the high-pressure feeding pump 12 at a flow velocity of 14g/min. The prepared mixture was passed through the heat-exchanger 13 to be delivered to the preheater 200 and pre-heated in the preheater 200 to a temperature of 280°C.

The pre-heated mixture was put into the reactor 300. A deoxygenation reaction and a functionalization reaction of the graphene are performed under a subcritical condition while maintaining an inner portion of the reactor at a temperature of 350°C and a pressure of 250atm. The product obtained after the reaction was completed was transferred into the heat exchanger 13 again and primarily cooled down at 200°C, and cooled down at 26°C through the cooling down system 15 again. The cooled resultant product was filtered by filtering parts 501 and 502. Here, the product was continuously washed while injecting distilled water by the distilled feeding pump 16, then the pressure was released to 1atm by pressure controlling systems 17 and 18, and the obtained graphene compound was recovered in product storing parts 511 and 512 including the filtering part. 3.9g of functionalized graphene was obtained by the continuous process.

### [Example 2]

The same process as Example 1 was performed except that the pre-heating temperature was 300°C, the reaction temperature was 380°C, and the reaction was performed in a supercritical condition, to obtain 3.5g of functionalized graphene in Example 2.

### [Comparative Example 1]

10g of the graphite oxide prepared by Preparation Example was heat treated in an electric furnace of 300 to 400°C for 2 minutes to obtain 8.4g of graphene.

Reaction conditions were summarized and shown in the following Table 1.

**[Table 1]**

| Classification | Reaction Condition | Graphite Oxide (g) | Functional Compound (g) |
|---|---|---|---|
| Preparation Example | - | - | - |
| Example 1 | Subcritical Water | 5 | 23.3 |
| Example 2 | Supercritical Water | 5 | 23.3 |
| Comparative Example 1 | - | 10 | - |

Contents of carbon and oxygen of the graphite oxide prepared by Preparation Example, and the graphene prepared by Example and Comparative Example were measured by elemental analyzer (EA).

Meanwhile, oxygen of the graphite oxide (5g) used in Example had a content of 40.1%(2.005g), that is, 0.125mol, and a molar ratio of the used functional compound (23.3g, 0.306mol) to the oxygen was 2.45.

The amount of the obtained graphene, the contents of each element of the graphene, and the molar ratio of the used functionalized compound to the oxygen included in the graphite oxide were summarized in the following Table 2.

**[Table 2]**

| Classification | Amount of Graphene (g) | Content of Carbon [wt%] | Content of Oxygen [wt%] | Amount of Functionalized Compound to Oxygen (Molar Ratio) |
|---|---|---|---|---|
| Preparation Example | - | 48.5 | 40.1 | - |
| Example 1 | 3.9 | 88.8 | 3.0 | 2.45 |
| Example 2 | 3.5 | 85.9 | 5.8 | 2.45 |
| Comparative Example 1 | 8.4 | 72.9 | 21.0 | - |

Referring to Table 2, in Comparative Example 1, the largest amount of product to the graphite oxide used in the reaction was obtained. The reason is because reduction was not effectively performed in the graphene obtained by Comparative Example 1, such that a large content of oxygen was contained, and this may be confirmed from the content of oxygen atom of Comparative Example 1 which is significantly higher than those of Examples 1 and 2.

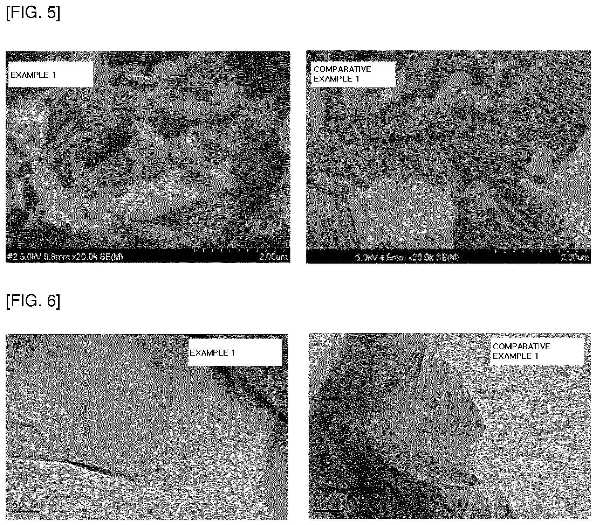

FIGS. 5 and 6 show a scanning electron microscope (SEM) and a transmission electron microscope (TEM) of the graphene obtained by Example 1 and Comparative Example 1 of the present invention, respectively.

Referring to FIGS. 5 and 6, it could be appreciated that in Comparative Example 1, the graphene was rarely exfoliated, a layered structure of the graphite was maintained as it is; meanwhile, in Example 1, the graphene was exfoliated from the graphite, such that the layered structure was rarely observed. That is, it could be appreciated that the graphene was more effectively exfoliated from the graphite and produced in Example 1 as compared to Comparative Example 1. It is considered that due to a steric hindrance effect of the functional group introduced into the surface or the edge of the graphene by the functional compound in Example 1, restacking phenomenon in which the graphene separated from the graphite is restacked to the graphite may be prevented, such that the exfoliation property of the graphene may be excellent.

### <Experimental Example>

### Raman Spectrum Measurement

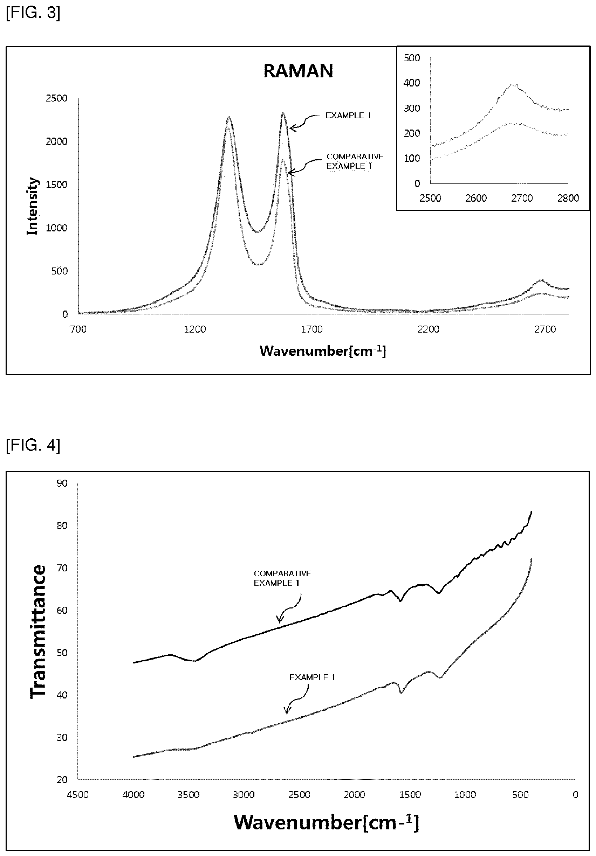

Raman spectrum was measured by using the graphenes obtained by Example 1 and Comparative Example 1, and results obtained by the measurement were shown in FIG. 3.

Referring to FIG. 3, it could be appreciated that in Example 1, peak was formed around 2700cm⁻¹ but the peak was rarely shown in Comparative Example 1. The peak is 2D peak indicating that the graphene was effectively exfoliated from the graphite, and degree of reduction was high, and therefore, it could be confirmed that high quality graphene was prepared in Example 1 as compared to Comparative Example 1.

### Infrared Spectrometry Spectrum Measurement

Infrared spectrometry spectrum was measured by using the graphene obtained by Example 1 and Comparative Example 1. Results obtained by the measurement were shown in FIG. 4.

Referring to FIG. 4, it was observed that a peak at 3300cm⁻¹ of hydroxy group, a peak at 1760cm⁻¹ of carbonyl group, a peak at 1130cm⁻¹ of C-O bond, and a peak of epoxy group shown in Comparative Example 1 were remarkably decreased in Example 1, such that it could be confirmed that reduction was effectively achieved in Example 1.

### XPS Measurement

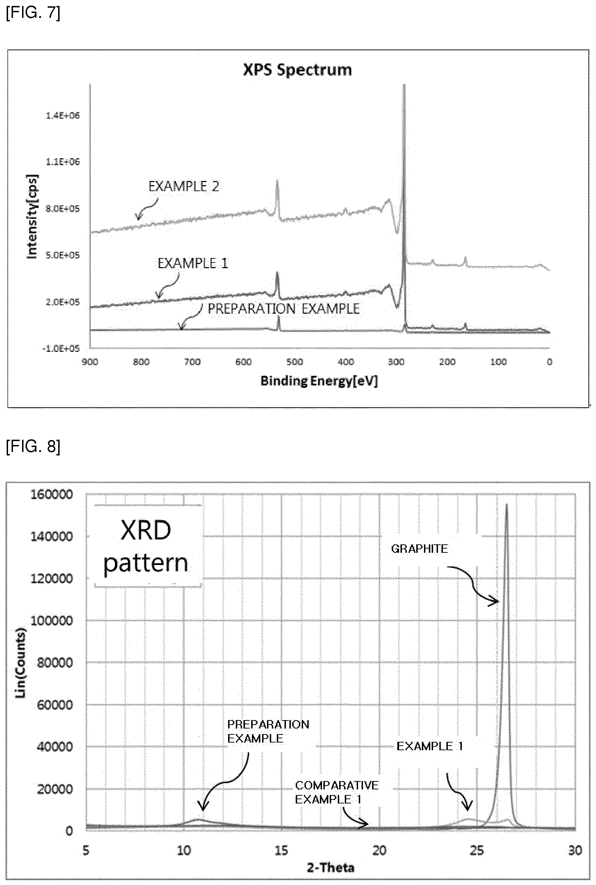

X-ray photoelectron spectroscopy (XPS) was measured by using the graphite oxide obtained by Preparation Example and the graphene obtained by Examples 1 and 2, and results obtained by the measurement were shown in FIG. 7.

Referring to FIG. 7, the introduction of the functional group including the sulfur into the surface and the edge of the graphene and the amount of the introduction could be confirmed. In Preparation Example, O₁ₛ(510∼540eV) peak was significantly high, and S₂ₚ(160∼170eV) peak was rarely detected; meanwhile, in Examples 1 and 2, O₁ₛ peak was remarkably low as compared to Preparation Example, and S₂ₚ peak was detected, and therefore, it could be confirmed that the functionalized graphene was obtained as a product.

In addition, as a result of XPS analysis, the sulfur contents in Examples 1 and 2 were calculated as 3.8wt% and 4.0wt%, respectively, and therefore, it could be confirmed that the functional group including the sulfur atom was introduced into the surface and the edge of the graphene by the functional compound.

### XRD Measurement

X-ray diffraction (XRD) was measured by using the graphite oxide obtained by Preparation Example and the graphenes obtained by Example 1 and Comparative Example 1, and results obtained by the measurement were shown in FIG. 8.

Referring to FIG. 8, it could be observed that 2θ = 11° peak which is a typical peak of the graphite oxide was rarely shown in Example 1, and a peak of a graphene crystal was shown in 2θ = 21∼26° corresponding to 0 0 2 plane peak of the graphene. Meanwhile, it could be appreciated that in Comparative Example 1, a decrease in a size of the 2θ = 11° peak was smaller than that of Example 1, such that the crystal structure of the graphene after reduction was not shown.

### [Description of Reference Numerals]

- 11:: circulation pump
- 12:: high-pressure feeding pump
- 13:: heat exchanger
- 14:: functional compound feeding pump
- 15:: cooling down system
- 16:: distilled water feeding pump
- 17,18:: pressure controlling system
- 100:: mixing part
- 200:: preheater
- 300:: reactor (or functionalizing reactor)
- 400:: cooling down and depressurizing system
- 501, 502:: filtering part
- 510, 511, 512:: product storing part

## Claims

1. A method for preparing a functionalized graphene comprising:
forming a mixture including a graphite oxide, water, and thiourea;
pre-heating the mixture to 200 to 400°C;
forming the functionalized graphene by reacting the mixture under a supercritical condition of the solvent; and
recovering the functionalized graphene.

2. The method of claim 1, wherein the solvent is included in an amount of 10 to 100,000 parts by weight based on 100 parts by weight of the graphite oxide.

3. The method of claim 1, wherein thiourea is included at 0.1 to 30 molar ratio with respect to oxygen included in the graphite oxide.

4. The method of claim 1, further comprising, after the forming of the functionalized graphene and before the recovering of the functionalized graphene, high-pressure-filtering the functionalized graphene.

5. The method of claim 1, further comprising, after the forming of the functionalized graphene and before the recovering of the functionalized graphene, washing the formed functionalized graphene.

## Patentansprüche

1. Verfahren zur Herstellung eines funktionalisierten Graphens umfassend:
Bilden einer Mischung, die ein Graphitoxid, Wasser und Thioharnstoff enthält;
Vorerhitzen der Mischung auf 200 bis 400 °C;
Bilden des funktionalisierten Graphens durch Umsetzen der Mischung unter einer überkritischen Bedingung des Lösungsmittels; und
Gewinnen des funktionalisierten Graphens.

2. Verfahren nach Anspruch 1, bei dem das Lösungsmittel in einer Menge von 10 bis 100.000 Gewichtsteilen, bezogen auf 100 Gewichtsteile des Graphitoxids, enthalten ist.

3. Verfahren nach Anspruch 1, bei dem Thioharnstoff in einem Molverhältnis von 0,1 bis 30 in Bezug auf in dem Graphitoxid enthaltenen Sauerstoff enthalten ist.

4. Verfahren nach Anspruch 1, ferner umfassend Hochdruckfiltration des funktionalisierten Graphens nach dem Bilden des funktionalisierten Graphens und vor dem Gewinnen des funktionalisierten Graphens.

5. Verfahren nach Anspruch 1, ferner umfassend Waschen des gebildeten funktionalisierten Graphens nach dem Bilden des funktionalisierten Graphens und vor dem Gewinnen des funktionalisierten Graphens.

## Revendications

1. Procédé de préparation d'un graphène fonctionnalisé comprenant les étapes consistant à :
former un mélange incluant un oxyde de graphite, de l'eau et thiourée ;
préchauffer le mélange entre 200 et 400 °C ;
mettre en forme le graphène fonctionnalisé par réaction du mélange dans des conditions supercritiques du solvant ; et
récupérer le graphène fonctionnalisé.

2. Procédé selon la revendication 1, où le solvant est inclus dans une proportion de 10 à 100 000 parts en masse par rapport à 100 parts en masse de l'oxyde de graphite.

3. Procédé selon la revendication 1, où la thiourée est incluse dans un rapport molaire de 0,1 à 30 par rapport à l'oxygène inclus dans l'oxyde de graphite.

4. Procédé selon la revendication 1, comprenant en outre, après la formation du graphène fonctionnalisé et avant la récupération du graphène fonctionnalisé, l'étape consistant à filtrer à haute pression le graphène fonctionnalisé.

5. Procédé selon la revendication 1, comprenant en outre, après la formation du graphène fonctionnalisé et avant la récupération du graphène fonctionnalisé, l'étape consistant à laver le graphène fonctionnalisé formé.
